# EUROPEAN PATENT SPECIFICATION

(11) **EP 2 004 840 B1**
(45) Date of publication and mention of the grant of the patent: **29.10.2014**
(21) Application number: 07736103.8
(22) Date of filing: 13.04.2007
(51) Int. Cl.: G01N 33/543, C12Q 1/00, G01N 33/84

(54) **SENSOR COMPRISING CONDUCTING POLYMER MATERIALS**
SENSOR MIT LEITENDEN POLYMERMATERIALIEN
CAPTEUR COMPRENANT DES MATÉRIAUX POLYMÈRES CONDUCTEURS

(30) Priority: 13.04.2006 IE 20060288
(43) Date of publication of application: 24.12.2008
(73) Proprietor: Dublin City University, Dublin 9 (IE); UNIVERSITY OF WOLLONGONG, Wollongong, NSW 2522 (AU)
(72) Inventor: MORRIN, Aoife, Dublin 15 (IE); SMYTH, Malcolm, County Meath (IE); KILLARD, Anthony, J., County Meath (IE); NGAMNA, Orawan, Prachinburi 25140 (TH); WALLACE, Gordon, George, New South Wales 2500 (AU); MOULTON, Simon, Edward, New South Wales 2525 (AU); CROWLEY, Karl, Dublin 3 (IE)
(74) Representative: O'Brien, John Augustine
(86) International application number: PCT/IE2007/000047
(87) International publication number: WO 2007/119229

(56) References cited:
- MORRIN A ET AL: "Novel biosensor fabrication methodology based on processable conducting polyaniline nanoparticles" ELECTROCHEMISTRY COMMUNICATION, ELSEVIER, AMSTERDAM, NL, vol. 7, no. 3, March 2005 (2005-03), pages 317-322, XP004741129 ISSN: 1388-2481 cited in the application
- MORRIN A. ET AL.: "An amperometric enzyme biosensor fabricated from polyaniline nanoparticles" ELECTROANALYSIS, vol. 17, no. 5-6, 2005, pages 423-430, XP002442159
- MOULTON S.E. ET AL.: "Polymerisation and characterisation of conducting polyaniline nanoparticle dispersions" CURRENT APPLIED PHYSICS, vol. 4, 2004, pages 402-406, XP002441975
- MOON GYU HAN ET AL.: "Preparation and characterization of polyaniline nanoparticles synthesized from DBSA micellar solution" SYNTHETIC METALS, vol. 126, 2002, pages 53-60, XP002442163
- JIAXING HUANG ET AL.: "The intrinsic nanofibrillar morphology of polyaniline" CHEMICAL COMMUNICATIONS, 2006, pages 367-376, XP002442169 cited in the application
- JOURNAL OF METASTABLE AND NANOCRYSTALLINE MATERIALS: "Ag Dispersed Conducting Polyaniline Nanocomposite as a Selective Sensor for Ammonia", JOURNAL OF METASTABLE AND NANOCRYSTALLINE MATERIALS, TRANS TECH PUBLICATIONS LTD, UETIKON-ZURICH, CH, vol. 23, 1 January 2005 (2005-01-01), pages 323-326, XP009149833, ISSN: 1422-6375

## Description

### Introduction

Conducting polymer materials are a unique class of organic materials that exhibit electrical and optical properties of metals and semiconductors (Saxena & Malhotra, 2003). They offer prospects for practical applications due to their levels of conductivity, inexpensiveness, and ease of synthesis. Among all of the conducting polymers, polyaniline is probably the most widely studied because it has a broad range of tunable properties derived from its structural flexibility. It has potential in numerous applications including sensors, rechargeable batteries, light-emitting diodes, corrosion protection of metals and gas separation membranes. Yet, like many other electrically conducting polymers, polyaniline has proved difficult to exploit for a number of reasons. It is insoluble in common solvents which seriously hinders its processability. The monomer, aniline, is a carcinogen. It must be distilled prior to use and stored under nitrogen. Finally, acidic conditions are required for the formation of the most highly conductive form of polyaniline (PANI), which does not lend itself to entrapment of pH-sensitive materials such as proteins (Morrin et al., 2005).

To date, the application of conducting polymeric materials is limited to low-speed areas. Several techniques have been employed in fabrication of polymer thin films, such as thermal evaporation, electropolymerisation (Grennan et al., 2005), spin-coating (Fujii et al., 2005), dipping (Zhang et al., 2002), and electrophoretic patterning (Li et al., 2005).

Conducting polymers are attractive for electronic applications and using inkjet printing, ultra-thin films can be patterned with resolution up to 20-30 µm (Calvert, 2001). Chen et al., 2003 describes inkjet printing an all-polymer RC filter circuit using soluble conducting polyaniline and poly(3,4-ethylenedioxythiophene). US Patent No. 6,576,975 to Yang et al. discloses an organic semiconductor device that employed inkjet printing for patterning high quality 'buffer' layers of conducting polymer between upper and lower electrodes to regulate current flow between the electrodes. Other devices fabricated by inkjet printing of conducting polymers in the electronics field include thin-film transistors (Paul et al., 2003), transistor circuits (Sirringhaus et al., 2000) and a chemical fuse (Mabrook et al., 2005). US Patent No. 6,501,587 to Ferraris et al. discloses electrochromic displays that have been fabricated using conducting polymer materials in conjunction with inkjet printing technology.

US Patent No. 6,762,050 to Fukushima et al. discloses the manufacture of a microsensor device where soluble conducting polymers such as polypyrrole, polythiophene and polyaniline were inkjet printed between two microelectrodes, and used for the detection of biomolecular or other organic species.

Inkjet printing of conducting polymers described to date relies on soluble conducting polymer which has inherent disadvantages. When the polymer is soluble in water, nonaqueous solvents must be used for the sensor application (Ballarin et al., 2004), or alternatively, a membrane must be applied on top in order to prevent dissolution of the films in the aqueous buffer solutions that are employed (Setti et al., 2005). If a polymer is soluble in organic media, incorporation of protein is not favourable. In addition, inkjet printing is very vulnerable to blockage due to solvent drying and hence a printing method that relies on rapid evaporation of solvent can be intrinsically difficult to maintain (Calvert, 2001). US Patent No. 6,762,050 discloses that if the polymer is insoluble in both common organic and water-based solvents, the polymer needs to be synthesised directly on the substrate, wherein monomer is printed onto oxidant-containing films.

Prior art publications are limited to water- or organic-soluble conducting polymers such as doped polyaniline derivatives or Poly(3,4-ethylenedioxythiophene) poly(styrenesulphonate) (PEDOT/PSS). Films cast from water-soluble materials are generally unstable in aqueous media, and hence often require the use of additional materials, such as polycations, in order to render the polymer insoluble (Ngamna et al., 2005).

Alternate types of nanoparticles, primarily metallic in nature are also being exploited for both electrochemical and optical sensing applications. Published US Patent Application No. US2006014005 discloses a sensing device fabricated from metallic nanoparticles that may be deposited by inkjet printing. Published patent application No.s EP 1 608 975 and IE20040203 disclose the use of silver metallic nanoparticles for sensing applications, primarily optical sensing.

Athawale and Katre (Journal of Metastable and Nanocrystalline Materials, Vol. 23, 1 January 2005, pages 323-326) describe the use of a silver polyaniline nanocomposite as a sensor for ammonia vapors.

Therefore, the development of a new technique to selectively pattern conducting polymers is necessary for their realisation as electrodes in electronic devices.

### Statements of Invention

The invention provides use of an electrode having nanoparticles of polyaniline inkjet printed thereon as an electrochemical sensor for the detection of ammonia.

The nanoparticles may be substantially spherical in shape. The size distribution of the nanoparticles may be in the range of from 1nm to 100nm, such as in the range of from 10nm to 50nm. Alternatively, the size distribution of the nanoparticles may be in the range of from 20 to 80nm, typically 30nm to 80nm.

The nanoparticles may be inkjet printed onto the electrode using piezoelectric technology.

The sensor may be used for chemical sensing.

The sensor may be used for direct sensing of ammonia.

The electrode may be a thermostable material for example a thermostable material that may undergo dynamic recovery upon the application of heat.

Also described is a printing composition for printing onto a substrate, the composition comprising nanoparticles of a conducting polymer. The composition may be an inkjet composition for inkjet printing onto a substrate.

The nanoparticles of the composition may be substantially spherical in shape.

The size distribution of the nanoparticles of the composition may be in the range of from 1nm to 100nm. For example, in the range of from 10nm to 50 nm. Alternatively, the size distribution of the nanoparticles may be in the range of from 20nm to 80nm, typically 30nm to 80nm

Also described is a method for obtaining a pattern on a substrate comprising printing nanoparticles of a conducting polymer onto the substrate. An ink containing nanoparticles of a conducting polymer may be printed onto a substrate. the nanoparticles may be formed of polyaniline. The printing may be inkjet printing.

The nanoparticles may be substantially spherical in shape. The size distribution of the nanoparticles may be in the range of from 1nm to 100nm, such as in the range of from 10nm to 50 nm. For example, in the range of from 20nm to 80nm, typically 30nm to 80nm.

Also described are nanoparticles of a conducting polymer, the nanoparticles being substantially spherical in shape. The diameter distribution of the nanoparticles may be in the range of from 1nm to 100nm, such as from 10nm to 50nm for example, 20 to 80 nm, typically 30nm to 80nm. The nanoparticles may be formed of polyaniline.

Also described is a method of regenerating a sensor comprising a substrate having nanoparticles of a conducting polymer printed thereon, the method comprising applying heat to the sensor. The heat source may be a stream of heated air. The heat source may be applied directly to the sensor.

Also described is a method of sensing hydrogen peroxide comprising the steps of:
- setting up a three electrode cell comprising a reference electrode, an auxiliary electrode and a working electrode wherein the working electrode has nanoparticles of a conducting polymer printed thereon;
- adding an electrolyte solution;
- applying a potential of about 100mV vs the reference electrode to the working electrode and allowing the resulting current to reach a steady state;
- adding hydrogen peroxide to the cell; and
- monitoring the amperometric response.

The auxiliary electrode may be platinum. The reference electrode may be Ag/AgCl. The nanoparticles may be formed of polyaniline. The electrolyte solution may be phosphate buffered saline pH6.8.

### Brief Description of the Drawings

The invention will be more clearly understood from the following description thereof given by way of example only in which:-
Fig. A is a schematic representation of a polyaniline nanoparticle synthesised according to Example 1 by the dodecylbenzenesulphonic acid (DBSA) micellar system, where after dialysis, there should be substantially no monomer remaining;
Fig 1A is a line graph depicting the % volume vs. diameter of a dispersion of polyaniline nanoparticles, synthesized according to Example 1, with an average size of approximately 40nm;
Fig 1B is a line graph depicting the % volume vs. diameter of a dispersion of polyaniline nanoparticles, synthesized according to Example 1, with an average size of approximately 70nm;
Fig 2 is a transmission electron microscopy (TEM) image of polyaniline nanoparticles synthesised according to Example 1, the image was obtained at 100 keV and at a magnification of x 200k;
Fig. 3A is a 3-D profilometry of an inkjet printed film (Example 3) of the polyaniline nanoparticles (Example 1) on Polyethylene Terephthalate (PET) (Example 2D) comprising 24 bi-directional prints;
Fig 3B is a 3-D profilometry of a 5 µl drop-coated film of polyaniline nanoparticles (Example 1) on PET (Example 2D);
Fig. 4A is a scan of Atomic Force Microscopy (AFM) analysis of a bare gold mylar substrate (scan size 1µm);
Fig. 4B is a scan of Atomic Force Microscopy (AFM) analysis of inkjet printed (Example 3) polyaniline nanoparticles (Example 1) (1 print) on gold mylar (scan size 1µm) (Example 2E);
Fig. 4C is a scan of Atomic force microscopy (AFM) analysis of inkjet printed (Example 3) polyaniline nanoparticles (Example 1) (10 prints) on gold mylar (scan size 1µm) (Example 2E);
Fig. 5A is a graph showing a scan rate study in 1M HCl of an inkjet printed polyaniline nanoparticle film, comprising 24 bi-directional prints, on a screen-printed carbon paste electrode (Example 3a);
Fig. 5B is a line graph showing the dependency of the peak current of peaks I and II of Fig 5A on scan rate;
Fig. 5C is a line graph showing the dependency of the peak current of peaks I and II of Fig 5A on (scan rate)^{1/2};
Fig. 6A is a cyclic voltammogram (n=5) of inkjet printed polyaniline films (50 prints) in the mono (^{_______}) and bi directional modes (-------);
Fig 6B is a bar chart of the % RDS for peak I of Fig. 6A for inkjet printed polyaniline films in both the mono (black) and bi directional modes (grey);
Fig. 7 is a cyclic voltammogram comparison of the electrochemistry of an inkjet printed film (50 prints) and a 5 µl drop-coated film on screen printed electrodes (cyclic voltammetry was carried out in 1 M HCl at 500 mV s⁻¹);
Fig. 8 is a graph showing a rheological study of the polyaniline nanoparticle dispersions and a commercial Epson ink (T038);
Fig. 9A is a graph showing amperometric response of ammonium ions at a concentration of 51, 17, 9 and 4 ppm respectively in 0.1 M phosphate buffer pH 6.6, -0.4 V vs. Ag/AgCl;
Fig. 9B is a graph showing amperometric response of ammonium ions at a concentration of 2, 1 and 0.25 ppm respectively in 0.1 M phosphate buffer pH 6.6, -0.4V vs. Ag/AgCl;
Fig. 10A, B and C are line graphs showing amperometric data for inkjet printed films of polyaniline nanoparticles (50 prints) on screen-printed carbon paste electrodes in 0.1 M Phosphate buffer pH 6.6 and a potential of -0.4V vs. Ag/AgCl;
Fig. 11 is a photographic representation of a 25 layered inkjet printed polyaniline nanoparticle film on a screen-printed silver interdigitated array (IDA) electrode. The numbers at the base (200 x 1500) refer to the digit width (200µm) and digit space (1500µm);
Fig. 12 is a current-potential plot obtained when a varying potential is applied to a PANI-IDA;
Fig. 13A is a plot showing the raw current-potential response (as recorded) of a PANI-IDA exposed to ammonia gas;
Fig. 13B is a plot of current-potential response of PANI-IDA exposed to ammonia gas where the current was sampled at ±0.5V vs. time (cycle is 20 s);
Fig 14A is a graph showing the effect of ambient heat on electrode response (in an atmosphere free of ammonia) of a PANI-IDA electrode exposed to temperatures of between 20 to 120°C;
Fig 14B is a graph showing the effect of ambient heat on electrode response (in an atmosphere free of ammonia) of a PANI-IDA electrode exposed to temperatures of between 20 to 160°C;
Fig. 15 is a graph showing the effect on the response of a PANI-IDA electrode following long term exposure to a temperature of 75°C (the electrode was removed from the oven for 10 minutes prior to each measurement);
Fig. 16A and B are graphs showing the response (A) and recovery (B) of a 25 layer PANI-IDA sensor when exposed to vapour from a 0.25% ammonia solution with a concentration of approximately 60ppm;
Fig. 17 is a graph showing headspace analysis of ammonia gas using a PANI-IDA sensor with 25 inkjet prints at a digit width of 200µm and a digit space of 1500µm. The main graph shows the plot of I₀ - I (wherein I₀ is the initial current and I is the measured current) vs. log of the ammonia concentration (measured with an Impulse XP ammonia probe). The insert graph shows the measured current vs. ammonia concentration;
Fig. 18A is a plot showing real-time amperometric responses for inkjet printed polyaniline nanoparticle films (20 prints) on screen-printed carbon paste electrodes to a plurality of additions of 8mM hydrogen peroxide, the potential was held at -100mV vs. Ag/AgCl;
Fig. 18B is a plot showing real-time amperometric responses for electropolymerised polyaniline film (10 cycles) on screen printed carbon paste electrodes to a plurality of additions of 8mM hydrogen peroxide, the potential was held at -100mV vs. Ag/AgCl;
Fig. 19 is a calibration curve of the inkjet printed polyaniline electrode (20 prints) for the detection of hydrogen peroxide (8 mM additions) in 0.1M phosphate buffer at -100 mV vs. Ag/AgCl (n=3);
Fig. 20 is an amperogram illustrating the binding of HRP to the inkjet printed polyaniline nanoparticle films. Binding begins at 800 s and becomes saturated at 1250 s. Buffer is then passed over resulting in a return to background steady state signal. At approximately 1400 s, hydrogen peroxide only was passed over resulting in a catalytic signal confirmed by the binding of horse radish peroxidase (HRP) to the electrode surface;

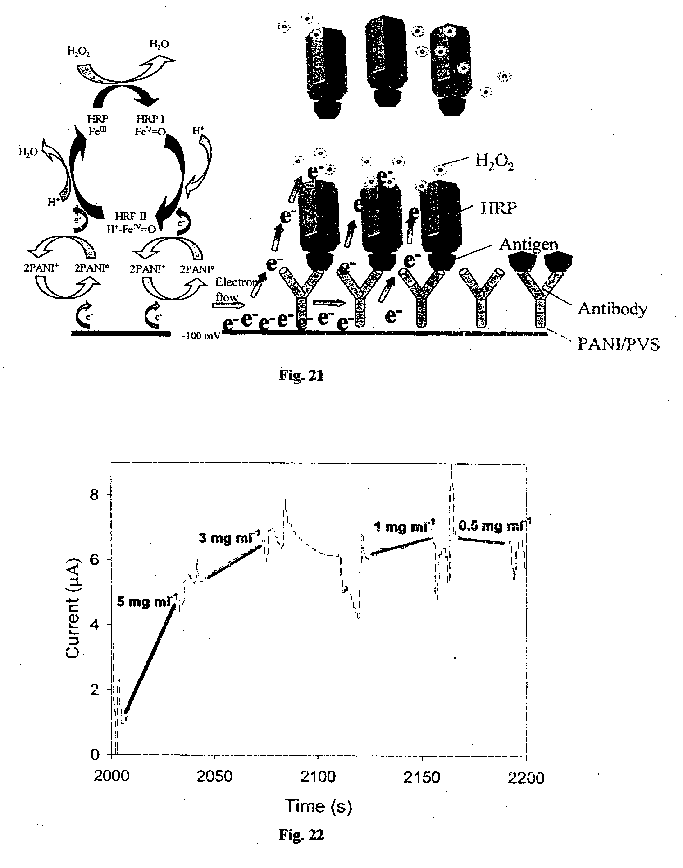

Fig. 21 is a schematic representation of an electrically wired immunosensor using polyaniline. The left hand schematic illustrating polyaniline (PANI⁺) being reduced by the electrode, HRP in its native state (HRP Fe^{III}) is reduced by hydrogen peroxide to become HRP^{I} (Fe^{V} = O). HRPI then undergoes two 1 electron reductions to HRP^{II} before returning to the native HRP state. Each of the 1 electron reductions results in the oxidation of PANI⁰. The right hand schematic illustrates a system that provides mediated electron transfer between the electrode and any surface-bound species present in the polymer, the system also acts as a point of electrostatic protein deposition.
Fig. 22 is a plot showing multi-calibrant analysis of HRP on a single polyaniline (Example 1) nanoparticle film inkjet printed (Example 3) onto a carbon paste, screen-printed electrode (Example 2A) surface. HRP standards (5 - 0.001 mg ml⁻¹) were passed over the electrode surface for 30 s with 1 M hydrogen peroxide. HRP bound to the electrode surface in real-time with the resulting changes in slope of the catalytic signal corresponding to changes in binding concentrations.

### Detailed Description

Improvements in synthesis and fabrication of conducting polymers with nanodimensional control have managed to overcome the issue of processibility. Little or no aniline monomer should be present in these nanoparticle dispersions. A stable nanodispersion has an indistinguishable appearance from a true solution, and more importantly can be handled and applied similarly (Li et al., 2005). In addition, enhanced properties of conducting polymer materials become apparent at the nanodimension such as higher conductivity and more rapid, discrete, electrochemical switching processes, properties directly applicable in electrode devices.

Nanoparticles of conducting polymers offer a route to overcome issues of solubility post-deposition. Once printed on a suitable substrate, nanoparticles adhere to the surface, and are rendered insoluble in mild media. In contrast, incorporation of water-soluble protein into the conducting polymer films (by printing, or otherwise), will, in the case of doped polyaniline, attach to the polymer and not be vunerable to detachment or dissolution in buffer. Not only do nanoparticles overcome vital processibilty issues, they possess numerous other advantages over their soluble counterparts, as described above, including higher conductivity and more rapid, electrochemical switching processes.

Using the conducting polymer nanoparticles with inkjet printing enables a practical route to a desktop fabrication system for sensing devices. This is demonstrated below using a nanoparticulate form of polyaniline. Ultra-thin films of polyaniline nanoparticles were patterned onto screen-printed electrodes using a conventional desktop Epson piezoelectric printer in conjunction with computer controlled software, Powerpoint™. The films were characterised using electrochemistry and physical techniques and an effective application in biosensing is demonstrated.

Inkjet printing may be performed with different ink ejection technologies, of which the most commonly used are the piezoelectric and thermal. Both thermal and piezo actuators accomplish the dispensing of pico-litre (pl) -sized droplets from the capillary nozzles of an inkjet printing head. In the thermal cartridge, the printhead is assembled in the ink cartridge. The printing head consists of a nozzle plate with several ink ejection orifices connected to resistive heater elements and placed in contact with an ink reservoir. When each individual heater element is actuated, rapid transfer of heat to a small portion of the ink located in the nozzles causes evaporation, creating a solvent bubble that leads to droplet formation. In piezoelectric actuators, the ink cartridge is detachable from the printhead. Crystalline materials in the printhead undergo mechanical stress upon application of an electric field. A very small contraction or expansion of these crystals, confined into the nozzles, allows for a reduction of space available for the ink, thus increasing pressure and causing ejection of the drop. Droplet sizes in both piezo and thermal technologies vary according to the temperature gradient applied, frequency, and ink viscosity.

### Example 1 - Synthesis of polyaniline nanoparticles

### Materials:

Aniline was purchased from Aldrich (13,293-4), vacuum distilled and stored frozen under nitrogen. Ammonium persulphate (APS), distilled aniline, dodecylbenzene sulphonic acid (DBSA) and sodium dodecylsulphonate (SDS).

### Method:

A modification of the rapid mixing method described by Huang, & Kaner (2006). was employed. 1.632 g of DBSA was dissolved in 16.75 ml water (0.25 M DBSA). 0.0032 mol of aniline (0.2982 g) and 0.0008 mol of APS (0.1826 g) were separately dissolved in 10 ml 0.25 M DBSA. Both solutions were mixed together in a vial. It was kept stirring for 2.5 hr. After polymerisation, 20 ml of 0.05 M SDS was added into the polymer dispersion. The solution was centrifuged at 4400 rpm for 30 minutes. The supernatant was decanted and put in a dialysis bag. It was dialysed against 0.05 M SDS (400 ml x 2) for 42 hours with the SDS solution being changed every 18 hours. Note: Dialysis is the separation of small solute particles from colloid particles by means of a semi-permeable membrane. Using a dialysis membrane with a cutoff point of 12,000 molecular weight (as in this instance), all species with molecular weights lower than this can be removed over time. Therefore, after dialysis for 42 hours, unreacted aniline monomer (93.13 molecular weight) would be completely removed

As the polymerisation begins, the initiator molecules (Ammonium Persulphate (APS)) induce the formation of nanoparticles by rapidly polymerising aniline monomers in the vicinity. If the initiator molecules are evenly distributed (by a rapid mixing in this instance), they should be consumed very quickly. Therefore secondary growth of polyaniline is very limited due to lack of available reagents. Carrying out the technique of. Huang & Kaner (2006) results in nanofibrillar formation. However, by carrying out the procedure in the presence of the micelle and dopant, dodecylbenzenesulphonic acid (DBSA), it is possible to stabilise the particles, as the reaction occurs in the hydrophobic core of the micelles, resulting in spherical nanoparticles with a diameter distribution between 10 and 50 nm or 20 and 80 nm.

Figure A is a schematic representation of a polyaniline nanoparticle synthesised in the DBSA micellar system as described in Example 1.

### Materials for Examples 2, 3 & 4

Polyaniline nanoparticles were synthesised as described in Example 1. Horseradish peroxidase (HRP, 232-668-6) was purchased from Biozyme Laboratories (South Wales, UK). 30% (v/v) hydrogen peroxide solution was purchased from Merck. Polyvinylsulphonate (PVS, 27,842-4) was purchased from Aldrich. The silver/silver chloride (Ag/AgCl) reference electrode was purchased from Bioanalytical Systems Ltd. (Cheshire, UK). The platinum mesh auxiliary electrode (29,809-3) was purchased from Aldrich. Gwent (C10903D14) carbon paste ink was purchased from Gwent Electronic Materials Ltd. (Gwent, UK). Electrodag^{®} PF-410S silver ink and Electrodag^{®} 452 SS BLUE insulation ink were purchased from Acheson Poly(ethylene) terephthalate (PET) substrates were purchased from HiFi Industrial Film Ltd (Dublin, Ireland). Indium Tin Oxide sputtered PET was purchased from CPfilms.

Unless otherwise stated, all electrochemical measurements were carried out in phosphate buffered saline (PBS), (0.1 mol dm⁻³ phosphate, 0.137 mol dm⁻³ NaCl and 2.7 mmol dm⁻³ KCl), pH 6.8.

### Instrumentation for Examples 2, 3 & 4

The ink-jet printing system was an Epson Stylus C45 printer. The chip resetter was purchased from www.9to6.ie. All electrochemical protocols were performed on a CH1000 potentiostat with CH1000 software, using either cyclic voltammetry or time-based amperometric modes. A DEK 248 screen-printer was used for fabrication of the carbon-paste and inter-digitated array electrodes.

### Example 2 - Substrates employed for inkjet printing, to include electrode substrates

**Example 2A** - Screen-printed electrodes were fabricated in-house using a DEK 248 according to Grennan et al. (2001). Briefly, electrodes were screen-printed onto pre-shrunk polyethylene terephthalate (PET) substrate. For the carbon paste electrodes: initially a layer of silver was deposited as the conducting path. A layer of Gwent carbon paste ink (C10903D14) was deposited as the working electrode. Finally, an insulation layer was deposited to eliminate cross-talk and to define the working electrode area (9 mm²).

**Example 2B:** Screen-printed electrodes were fabricated in-house using a DEK 248 according to Grennan et al. (2001). For the interdigitated array (IDA) electrodes: a layer of silver was screen-printed as the electrode layer onto pre-shrunk PET substrate. The resulting electrodes measured 30 mm from top to base and 24 mm across the base. A variety of pitch sizes were utilised, though the most commonly employed were IDAs with a digit width of 200 µm and a spacing of 1000 or 1500 µm. These dimensions were printed along the base of the electrode (i.e. 200 x 1500 for an IDA with 200 µm digits and 1500 µm spacing). The 200 x 1000 and 200 x 1500 IDAs had a total digit number of 17 and 12 respectively in an active area measuring approximately 20 x 20 mm.

**Example 2C:** Indium Tin Oxide sputtered PET

**Example 2D:** Bare PET

**Example 2E:** Gold sputtered PET

### Example 3 - Ink-Jet Printing of Nanoparticles

Epson print cartridges (T036 and T037), compatible with the Epson C45 were cut open and emptied of ink and the sponge inside was removed. All colour tanks of the cartridges (black, cyan, magenta and yellow) were cleaned thoroughly with deionized water. The chip on the cartridge was then reset using the chip resetter so that the printer would read the cartridge as full. Polyaniline nanoparticle dispersions (Example 1) were then poured into a tank in the cartridge, e.g., yellow. All other tanks were filled with deionized water.

Powerpoint® was used to draw coloured circles (3 mm diam.). The design was printed in colour, e.g., yellow, on plain printing paper (210 mm x 210 mm). Screen-printed electrodes were then affixed to the printed page where the Powerpoint® circles were aligned with the electrode area. The colour cartridge was then removed from the printer and replaced with the polyaniline-filled cartridge. Polyaniline was then printed as many times as required, on the screen-printed electrodes using 'Best Photo' mode as the printer setting.

### Example 4 - Assay procedures

### Example 4A - Ammonia sensing (aqueous phase)

Electrodes modified with polyaniline nanoparticles (synthesised according to Example 1) were incorporated into a flow-cell set-up as described by Killard et al., 2001. A potential of -400 mV vs. Ag/AgCl was applied. Following the reaching of a steady state, ammonium chloride at varying concentrations was passed over the sensor at a flow rate of 400 µl min⁻¹ for 20 s and the amperometric outputs monitored.

### Example 4B - Ammonia sensing (gas phase)

Interdigitated electrodes with polyaniline nanoparticles (synthesised according to Example 1) were incorporated into a gas jar for headspace analysis. An Impulse XP (Honeywell Analytics) ammonia sensor was employed as the reference method. Ammonia solution was introduced into the chamber dropwise. The polyaniline interdigitated electrode was connected in a 2 electrode configuration and cyclic voltammetry was applied (+0.1 V to -0.1 V at 0.2 V s⁻¹, cycled for duration of analysis). The current was sampled at +0.1 V and -0.1 V and plotted against time, yielding an i-t plot.

### Example 4C - Hydrogen Peroxide Sensing

Electrodes modified with polyaniline nanoparticles (synthesised according to Example 1) were incorporated into a batch-cell set-up as described by Killard et al., 2001. A potential of -100 mV vs. Ag/AgCl was applied. Following the reaching of a steady state, a number of additions of H₂O₂ (8 mM) were added to the cell, and the amperometric outputs monitored.

### Example 4D - Biosensing

Electrodes modified with polyaniline nanoparticles (synthesised according to Example 1) were incorporated into a flow-cell set-up as described by Killard et al., 2001. A potential of -100 mV vs. Ag/AgCl was applied. Following the reaching of a steady state, horseradish peroxidase (HRP) at varying concentrations from 5 to 0.01 µg ml⁻¹, were passed over with H₂O₂ (1 mM) at a flow rate of 400 µl min⁻¹ for 20 s and the amperometric outputs monitored.

### Results and Discussion

### Characterisation of particles (synthesised as per Example 1) in aqueous dispersion

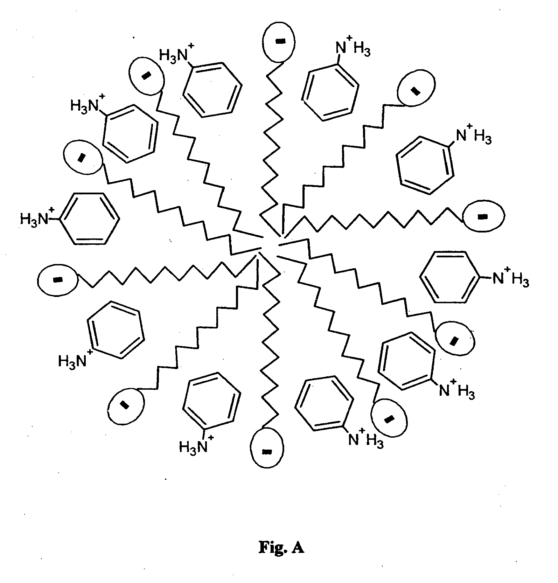

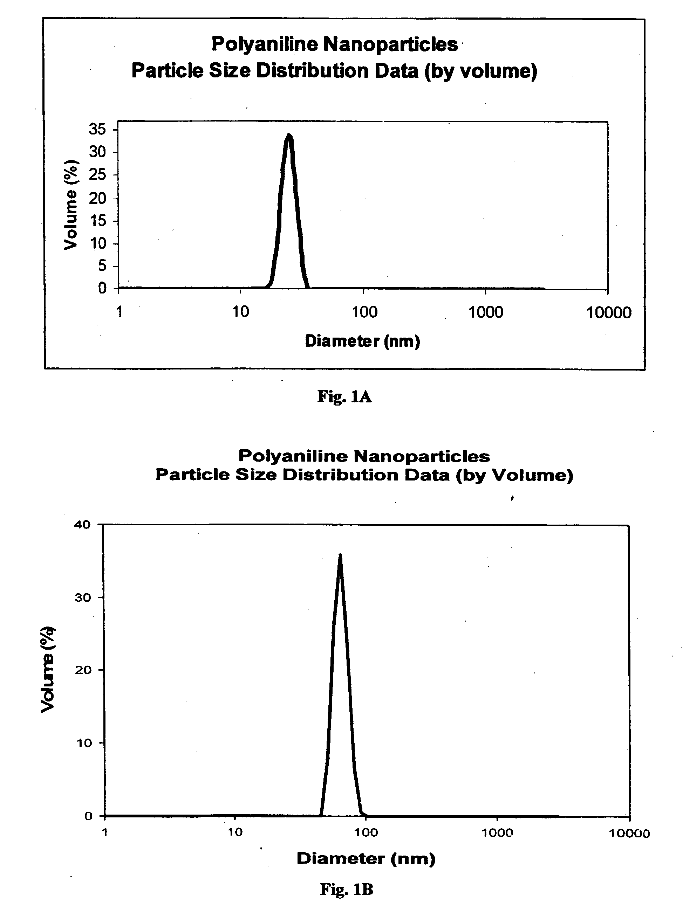

A critical property of the polyaniline nanoparticle dispersions (Example 1) is the particle size in concentrated solution. Dynamic light scattering was used to determine the particle sizes with the size distribution shown in Figure 1a & b. This indicates that the particle sizes in the dispersions are in the range of 10-50 nm or 20 - 80 nm. There may have been conditions such as changes in physical conditions such as temperature or slight experimental differences to result in two distinctly different size ranges. Beyond these possibilities, it is not known why there are variations in particle size. From a printing perspective, the typical pigment particle size is 100-400 nm (Magdassi & Ben Mo She, 2003) which can be printed using ink-jet printer with the nozzle diameter ca. 30-60 µm (Le, 1998). Therefore, the particle size of polyaniline nanoparticles should be at least in the same order of magnitude to the typical pigment particle size or even smaller. Due to effects of polymer aggregation, particle sizes bigger than 1000 nm was deemed unsuitable due to the possibility of clogging the print head.

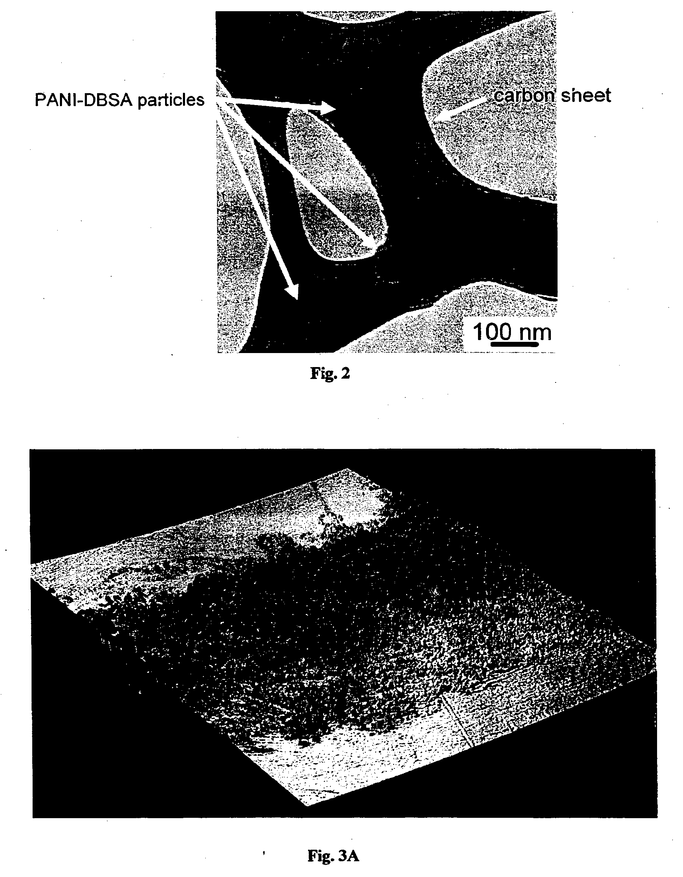

A Transmission Electron Microscope (TEM) image of the polyaniline nanoparticles shows that the emulsion polymerisation approach used results in spherical particle formation. The polyaniline nanoparticles were imaged on a carbon sheet where the particles were measured to be between 30 - 80 nm (Figure 2).

### Inkjet printing of polyaniline nanoparticles (methodology described in Example 3)

A desktop Epson printer that uses piezoelectric technology was used for printing the polyaniline nanoparticles (synthesised as per Example 1). Unlike thermal printers such as Hewlett Packard printers, heat is not required for droplet formation. Thermal printing uses 'shots' of heat at temperatures around 300°C. Although the flashtime of these shots are only just 2 µs, such high temperatures could induce thermal degradation of the printing material. Piezoelectric technology does not use heat, and does not degrade the material in any way. Another important advantage is more flexible ink formulations and substrates can be used in piezo technology over thermal printing.

Printing of polyaniline nanoparticles (2.3 % w/v) was carried out as described herein (Example 3) using an Epson Desktop C45 printer. In order to pattern a dispersion using the inkjet printing technique, particle size within the dispersion must be several orders of magnitude smaller than the nozzle of the inkjet printhead in order to prevent clogging and blockages of the nozzle. The diameter of the nozzle in Epson piezo printheads is reported to be of the order of 30 µm. The polyaniline nanoparticles used in this research have been demonstrated to have a diameters ranging between 1nm and 100nm, such as 10 and 50 nm, 20 and 80 nm, and 30 and 80nm according to Particle Size Distribution Analysis (PSD) and hence could easily be printed on an Epson printer. In addition, the nanoparticle dispersions contained the surfactants SDS and DBSA that helped prevent clogging of the nozzles if any aggregation of the nanoparticles did occur in the printhead. Both DBSA and SDS are present in the aqueous dispersion which, as surfactants will serve to decrease the surface energy of the dispersion. Whilst not wishing to be bound by theory, it is thought that this decrease in surface energy of the dispersion assists with the piezoelectric printing and the spreading of the ink on a substrate as it is deposited to form a homogenous film.

A major advantage of the nanoparticle dispersions that was exploited was their insolubility in aqueous media despite being deposited from an aqueous dispersion. This results in an environmentally friendly, facile method to deposit films that can be exploited for solvent sensitive applications such as bio- or immunosensing. However, it was demonstrated that although the deposited films were insoluble, the films were not stable on all substrates. Two conductive flexible substrates were employed for the printing of the nanoparticles - Indium Tin Oxide (ITO) (Example 2C) and screen-printed electrodes (Example 2A). The ITO-sputtered plastic had a smooth morphology and although the nanoparticles adhered well to the substrate, the films peeled off in aqueous media. This was thought to be due to combination of the surface properties of the ITO and the excess surfactant in the films. It was shown that when the ITO-modified films were washed gently with ethanol before being immersed in aqueous buffers, the remaining film adhered to the surface. However, this process impaired the quality of the doped polyaniline films and hence, they exhibited poor electrochemistry. Screen-printed carbon electrodes were also investigated as a substrate. The surface of screen-printed electrodes is extremely rough and were shown to be ideal for the printing of the nanoparticles. The nanoparticles adhered to the electrode surface, and were shown to be stable in aqueous media.

It is important to note that although this work is demonstrated using inkjet printing films of polyaniline nanoparticles, the nanoparticles themselves maybe deposited in a number of ways - for example, but not limited to, electrochemical deposition, dip-coating, drop-coating, spin-coating, inkjet and screen-printing and roll-to-roll printing.

### Physical Characterisation

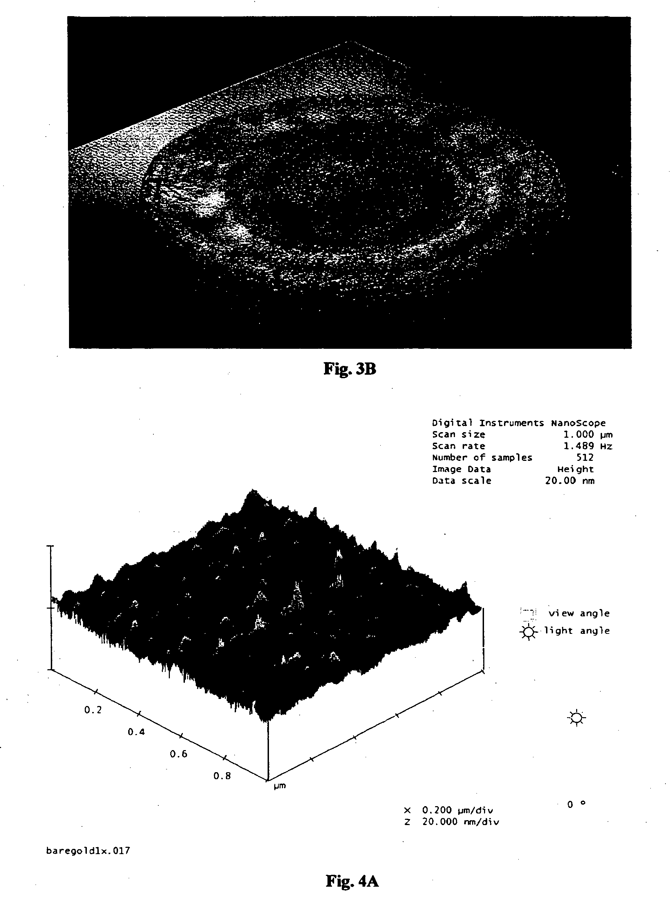

Profilometry was used to examine film morphology and thickness (Figure 3). The surface of the inkjet printed film appears quite rough, and composed of nodules of particles which the nanoparticles continuously deposit on as the film grows. Inkjet printed films (fabricated according to Example 3) on PET (Example 2D) substrate were compared to films drop-coated from 5 µl of dispersion. Inkjet printed films were shown to be highly homogeneous in nature (Figure 3a). Film thickness was measured to be 2.5 µm for 48 prints. Drop-coated films results in 'doughnut shaped' inhomogenous films (Figure 3b). The circumference of the film had a film thickness of 22 micron while the centre of the film was 10 times lower than that (2.2 micron). This mode of deposition from an aqueous medium therefore is much less controlled. In addition, they were shown to be more homogeneous. Figure 3a shows a 3-D profilometry image of an inkjet printed film on bare PET.

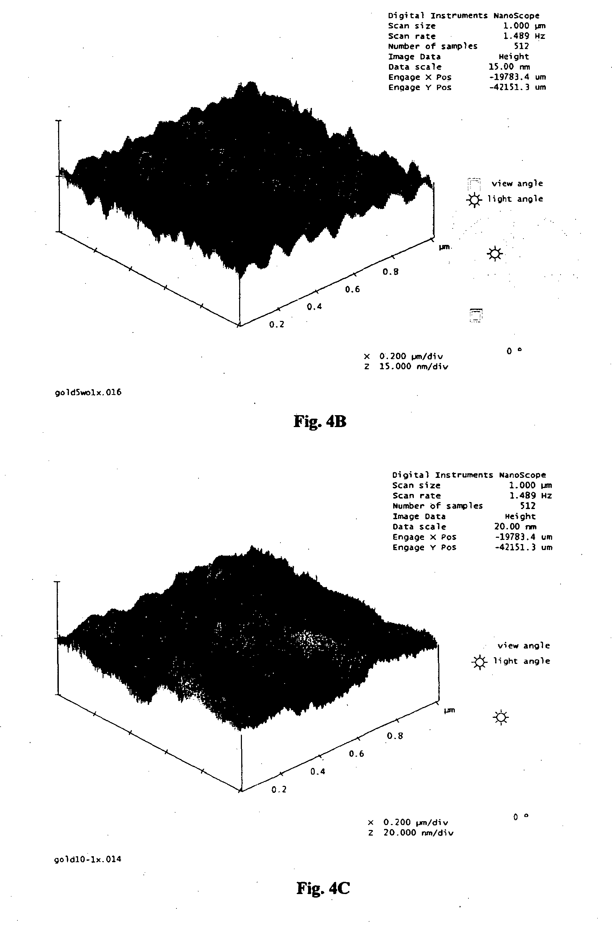

Atomic Force Microscopy (AFM) was used to investigate the resulting nanostructure of the inkjet printed polyaniline nanoparticulate films. Gold mylar was used as substrate (Example 2E) as the carbon-paste surfaces (Example 2A) proved to have too rough a morphology to visualise the nanoparticles. Figure 4a shows the morphology of the bare gold and the gold substrate modified with a single print of nanoparticles (Figure 4b) for a scan size area of 1 micron. It can be observed that the nanoparticles cover the background of the gold film. Figure 4c shows a film printed using 10 prints. The nodule size is much larger in this instance, probably due to increased aggregation upon drying in thicker films. Section analysis was also carried out. The individual nanoparticle sizes were measured in the different films. The maximum width of these nanoparticles is about 66 nm for 5 prints and 133 nm for 10 prints. These particle sizes are larger than what was measured by the PSD analysis, attributed to aggregation and increased agglomeration of the nanoparticles upon drying (i.e., removal of solvent). AFM tip distortion may also contribute.

### Electrochemistry of the ink-jet printed films

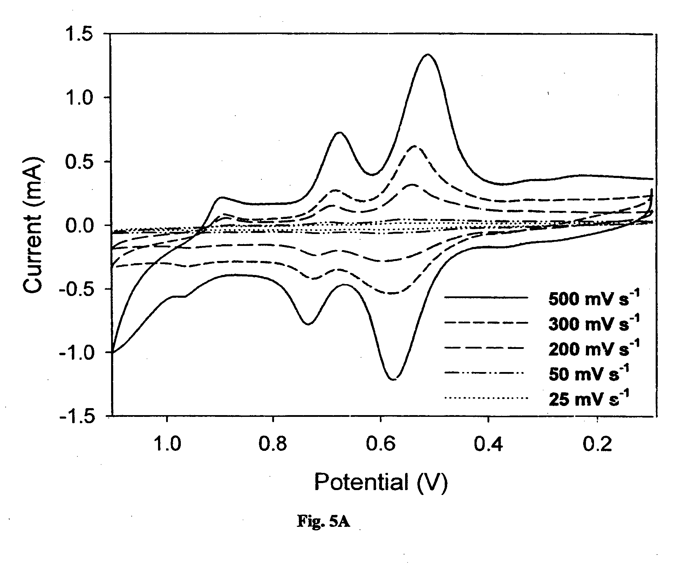

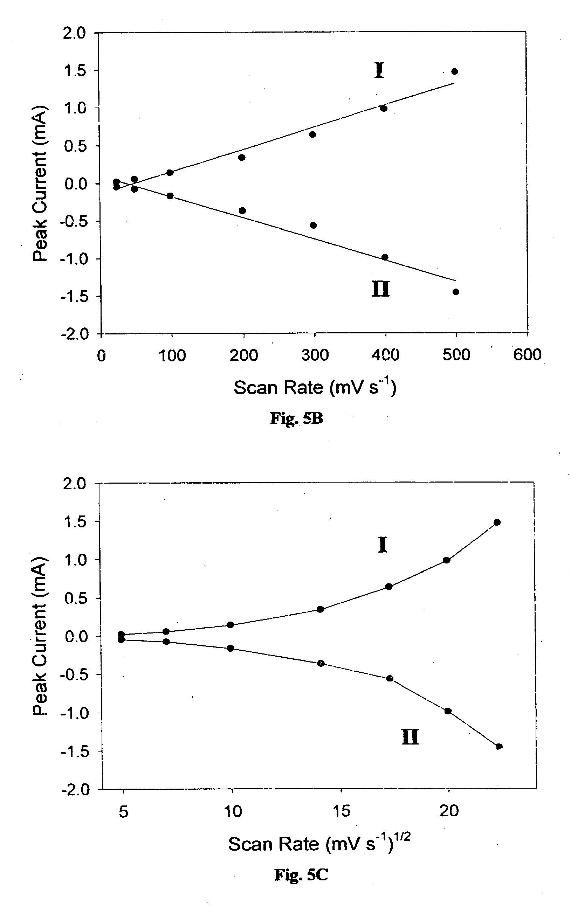

A cyclic voltammetric (CV) study of inkjet printed polyaniline films (Example 3) on carbon paste screen-printed electrodes (Example 2A) were carried out in HCl (1 M) (Figure 5a). The electrochemistry of the films were shown to be well-defined and stable, containing two primary sets of reversible peaks. Increasing the rate of change of potential causes the rate of electrolysis at the surface of the electrode to increase and results in increased peak currents. Using a single polyaniline-modified electrode, scan rates were varied from 25 mV s⁻¹ to 500 mV s⁻¹ and the relationship between the peak current and scan rate was investigated (Figure 5b). The linear relationship between the peak current and scan rate is in agreement with that of a thin film adsorbed electroactive species undergoing non-diffusional Nernstian reaction. This study also demonstrates a level of stability of the films on the screen-printed carbon paste electrode surface.

All desktop printers use mono-directional printing, where the printhead and the cartridge traverse across and back in the x-direction on a metal rod in a line recording operation, ejecting ink from nozzles according to the computer program it is in communication with. The substrate feeds out at a defined rate beneath the moving printhead, and takes the ink that is being printed. Using this mono-directional printing method, the nanoparticles were seen to be composed of lines, rather than as filled, homogeneous circles. In order to overcome this problem, a bi-directional printing method was employed, where the substrate was rotated 90° for every alternate print. This resulted in much higher coverage in the x and y direction of the surface of the electrode.

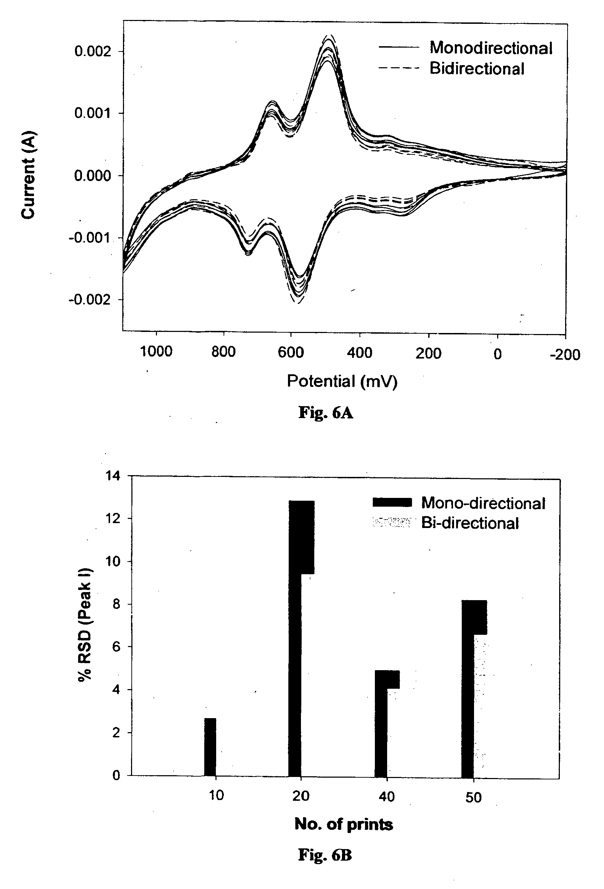

Cyclic voltammograms were generated for multilayer films printed in the mono- and bi-directional direction (Figure 6a). Five films of each type were fabricated and the data shows that there are is no overall difference in the CVs between mono- and bi-directional printing. % RSD values were obtained for Peak I for a number of prints (Figure 6b). It was shown that when films composed of 20 or more prints, the % RSD for each of the prints was significantly lower for bi-directional than mono-directional printing, possibly due to enhanced homogenity. As a result, all further printing was carried out in the bi-directional mode.

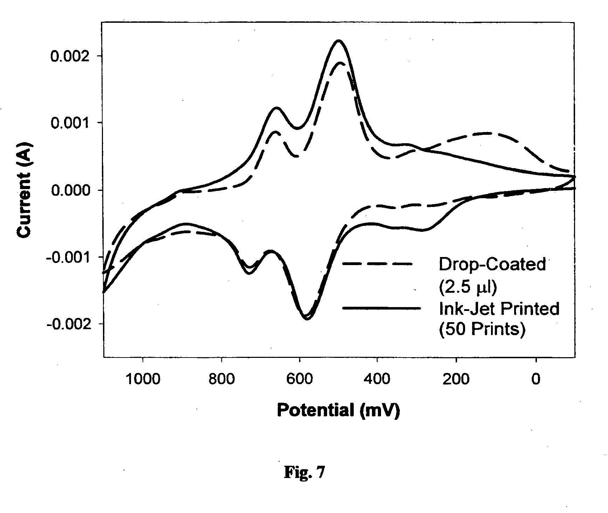

Electrochemistry was carried out on both types of films, and was shown to be comparable in terms of peak potentials. The inkjet printed film has however a CV that is much more defined at potentials lower than 400 mV. Although the inkjet printed films were estimated to be 10 times thinner than the bulk area of the drop-coated film, the peak currents of both films were approximately of equal magnitude. This allows us to estimate the volume of ink being deposited per inkjet print. Assuming 50 prints results in a film with an equivalent volume of 2.5 µl. This would mean that 50 nl are being deposited per print (Figure 7).

### Electrical Conductivity

A range of different ratios of aniline, oxidant and dopant were used for synthesis (methodology as per Example 1, however ratios of materials were varied) and resulting conductivity measurements of each of the cast polymer films (drop-coated onto PET, substrate example 2D) were performed using the four-point probe method (Table 1). The higher the proportion of APS used, the higher the conductivity obtained which could be attributed to a more efficient oxidation process during synthesis. Higher DBSA proportions resulted in lower conductivity due to the insulating effect of this surfactant. Incorporation of conducting materials such as gold nanoparticles or single-walled carbon nanotubes (SWNTs) into the polyaniline dispersions would help counteract the insulating effect of the DBSA and enhance the overall conductivity.

**Table 1. Bulk**

| **Formulation** | **Conductivity (mS cm⁻¹)** |
|---|---|
| 1.00:0.25:1.60 aniline:APS:DBSA (Example 1) | 0.8 ± 0.1 |
| 1.0:0.5:1.6 aniline:APS:DBSA | 2.2 ± 0.2 |
| 1.0:1.0:1.6 aniline:APS:DBSA | 32.8 ± 7.4 |
| 1.0:0.5:1.2 aniline:APS:DBSA | 4.3 ± 0.6 |

| | |
|---|---|
| conductivity measurements of polyaniline nanodispersions (n = 3). Note: Nanoparticle dispersions synthesised using different ratios of aniline:APS:DBSA (other than Example 1) have all been demonstrated to be inkjet printable, however the size of the resulting nanoparticles may vary, but are all within the 'inkjet printable' range i.e. less than 1000nm. | |

### Surface tension and rheological studies

The surface tension of each of the polyaniline nanodispersions in Table 1 were measured and compared to the Epson T038 black ink as shown in Table 2. All dispersions exhibited surface tensions in a range suitable for ink-jet printing (20-70 dyne cm⁻¹), although all were slightly below surface tensions of a commercial Epson ink (T038).

**Table 2. Surface tension data for polyaniline nanodispersions and commercial Epson T038 ink.**

| **Formulation** | **Surface tension (dyne cm⁻¹)** |
|---|---|
| 1.00:0.25:1.60 aniline:APS:DBSA (Example 1) | 27 ± 0.1 |
| 1.00:0.50:1.60 aniline:APS:DBSA | 27 ± 0.0 |
| 1.00:1.00:1.60 aniline:APS:DBSA | 29 ± 0.1 |
| 1.00:0.50:1.20 aniline:APS:DBSA | 28 ± 0.1 |
| Epson black ink (T038) | 30 ± 0.1 |

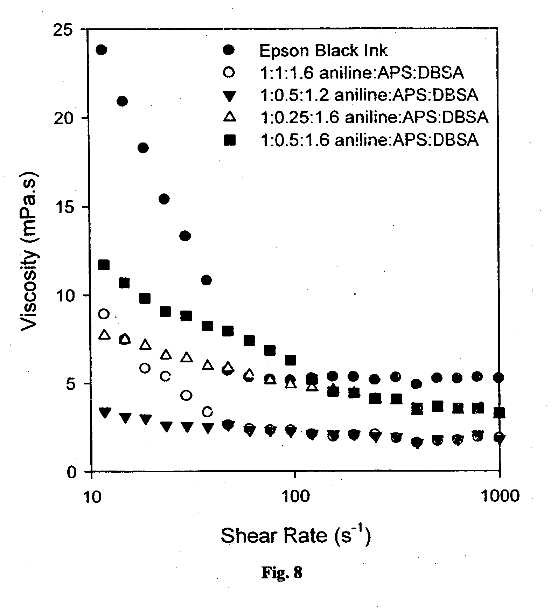

A rheological study of all of the polyaniline nanoparticle dispersions (including those synthesized according to Example 1) and the Epson T038 ink was also performed and the results are shown in Figure 8. At high shear rates (> 500 s⁻¹) the polyaniline nanoparticle dispersions and Epson T038 ink were comparable and behaved as Newtonian fluids. High shear rates are required inside the piezoelectric printhead nozzles (500 - 10⁵ s⁻¹ [13,14]). At lower shear rates, Epson T038 appears to be non-Newtonian in nature, exhibiting shear thinning which is an ideal ink property. The polyaniline inks deviate slightly from Newtonian, showing moderately higher viscosity at low shears. Moreover, the viscosity of all of the polyaniline nanoparticle dispersions are in the suitable range for inkjet printing (0.5-40 mPa s) although it was considerably lower than the viscosity of the Epson T038 ink when shear rates lower than 500 s⁻¹ were applied.

### Application of inkjet patterned films in ammonia sensing applications (Examples 4A &B

Conducting polymers, in particular, polyaniline, are beginning to emerge as excellent sensing materials for ammonia. The polymer is believed to be deprotonated by ammonia, which results in a measurable change in conduction. Inkjet printed films were examined as a potential sensing platform for detecting ammonium ions. Ammonia in equilibrium with ammonium ions, which comes from rain and snow. It is used frequently in refridgeration systems and needs to be monitored for effective cooling and for personal safety. Chemical fertilizers containing ammonia can stimulate the growth of plankton and be toxic for fishes. To prevent the water pollution, it is very important to monitor the level of ammonium ions. Furthermore, measurement of ammonium ions in biosensors has found an increasing application in the past 10 years because ammonium ions are a metabolic product in many enzymatic reactions. For example, ammonium and bicarbonate ions can be produced by the metabolism of urea with enzymatic action of urease. Therefore, urea can be detected by sensing its metabolic product, i.e., ammonium ions. Other applications for ammonia sensing are given in Table 3.

On exposure to ammonia, the polyaniline backbone is deprotonated. In the same way, certain amines such as dimethylamine, trimethylamine and triethylamine interact with the polyaniline, though the interactions will be influenced in part by the substituents on the nitrogen of the amine.

**Table 3. Requirements for ammonia analysis equipment in different application areas**

| **Application** | **Detection limit** | **Response time** | **Temperature range** | **Remarks** |
|---|---|---|---|---|
| **Environmental** | | | | |
| Monitoring ambient conditions | 0.1 ppb to >200 ppm | Minutes | 0-40 °C | Reduce environmental pollution |
| Measure in stables | 1 to >25 ppm | ∼1 min | 10-40 °C | Protect livestock animals and farmers |
| Monitoring in refridgerated spaces | 1 to 1000 ppm | Minutes | -40-0°C | pH range 7 ± 2 |

| **Automotive** | | | | |
|---|---|---|---|---|
| Measure NH₃ emission from vehicles | 4->2000 g/min (concentration unknown) | Seconds | Up to 300 °C | NH₃ emission is not regulated at this time |
| Passenger cabinet air control | 50 ppm | ∼1 s | 0-40 °C | Automotive air |
| Detect ammonia slip | 1-100 ppm | Seconds | Up to 600 °C | Control Urea injection in SCR NOx reduction |

| **Chemical** | | | | |
|---|---|---|---|---|
| Leakage alarm | 20->1000 ppm | Minutes | Up to 500 °C | Concentrations can be very high at NH₃ plants and can even be explosive |

| **Medical** | | | | |
|---|---|---|---|---|
| Breath analysis | 50-2000 ppb | ∼1 min | 20-40 °C | Diagnosis of peptic ulcer cause by bacteria, small gas volumes |

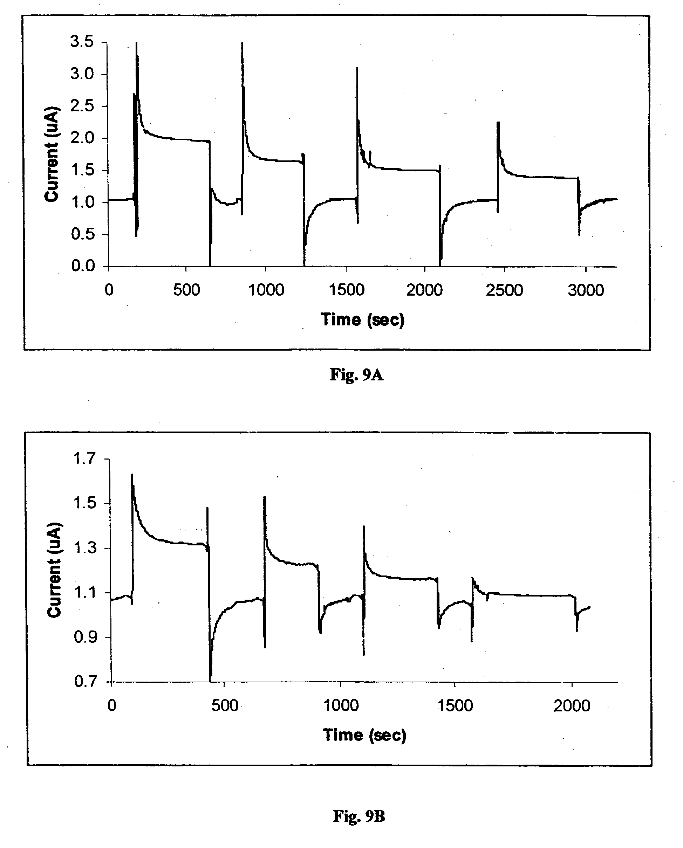

Inkjet printed films of polyaniline (Example 3) on a disposable screen-printed carbon-paste electrode platforms (Example 2A) provides a facile route to fabricating ammonia sensors. They can be mass-produced at extremely low cost and can provide comparable detection limits and linear ranges as commercial sensors based on metal oxides. Figure 9a & b are the amperometric responses of the inkjet printed polyaniline films (deposited on the carbon paste screen-printed electrode platform, Example 2A) to ammonium ions in solution using a flow injection setup (Example 4A). Using a flow rate of 400 µl min⁻¹, the response time is approximately 350 s at high concentrations of ammonia (50 ppm). At lower concentrations of ammonia, the response time decreases to about 150 s. With further optimisation, these response times could be further reduced. The flow rate determines the rate at which steady state conditions are established in the flow cell. Relative humidity may also effect the response time, and this was not investigated for these experiments.

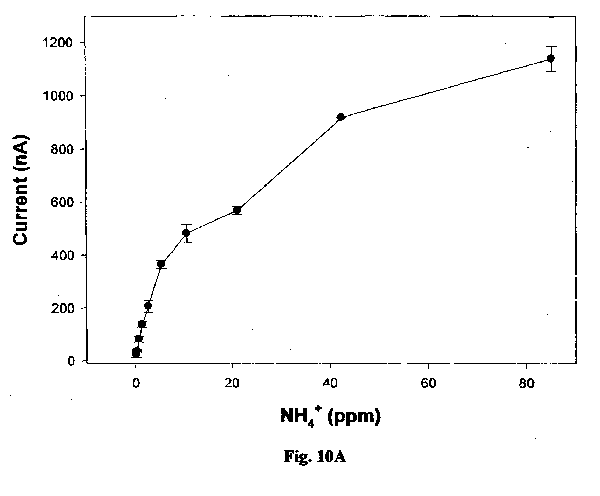

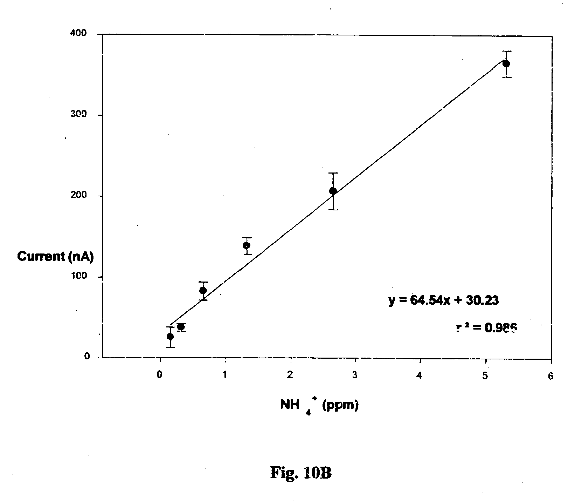

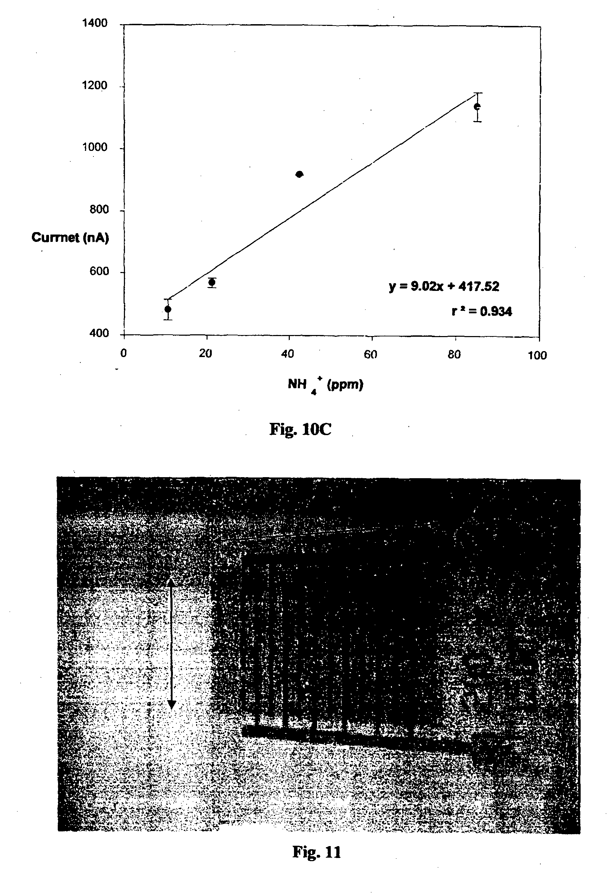

Figure 10a shows the amperometric responses for the inkjet printed polyaniline nanoparticle films on carbon-paste screen-printed electrodes to the ammonium ion from 0 to 85 ppm. The potential was held at -0.4 V vs. Ag/AgCl. The buffer used was a phosphate buffer (0.1 M, pH 6.6). Figures 10b & c show the two linear ranges for NH₄⁺ (0-5.3 ppm and 10 - 85 ppm) within that range.

Inkjet printed polyaniline films can also be used to detect ammonia in the gas phase (Example 4B). In this case the substrate/electrode is of the form of an Interdigitated Array (IDA); screen printed using silver ink (see Example 2B). The polyaniline nanoparticle film is inkjet printed as described in Example 3. Once printed, these polyaniline nanoparticle-modified IDAs were heat cured at 75°C for 30 minutes. Various configurations and dimensions of IDA can be employed and Figure 11 shows an example of the silver IDA electrode with a 25 layer polyaniline nanoparticle film inkjet printed over it (25 x 14 mm dimension, in this example). This configuration will be referred to as a PANI-IDA electrode from here on. The PANI-IDA electrodes are used in a 2-electrode cell configuration (i.e. anode and cathode). At a particular potential, the current passed between the electrodes is dependent on the conductivity of the film.

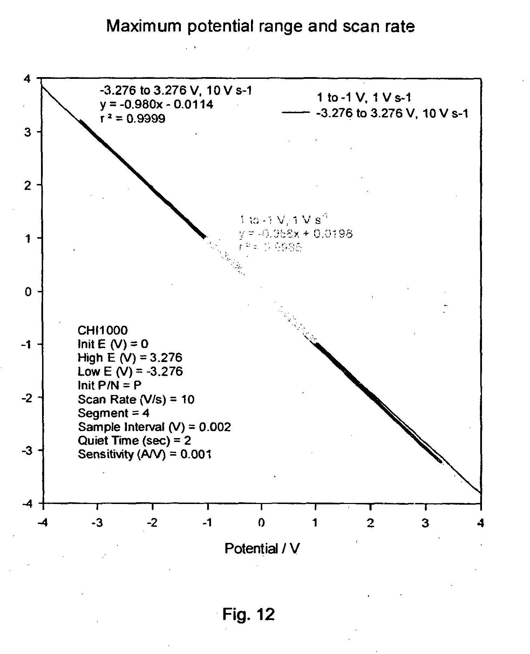

Applying a fixed potential between the electrodes, results in a gradual decrease in conductivity under ordinary atmospheric conditions, presumably due to a change in the oxidation state of the polymer to a non-conducting form. This effect can be countered by applying a waveform to the electrode (i.e. saw-tooth as applied in cyclic voltammetry). Figure 12 shows the current response obtained for varying potential. The slope of the plot (related to the conductivity of the film) is independent of scan rate. The saw-tooth plot results in a linear relationship between potential and current - implying Ohmic behaviour is being obeyed. Unlike in the case of applied fixed potential, the PANI-IDA displays stable behaviour over the long term.

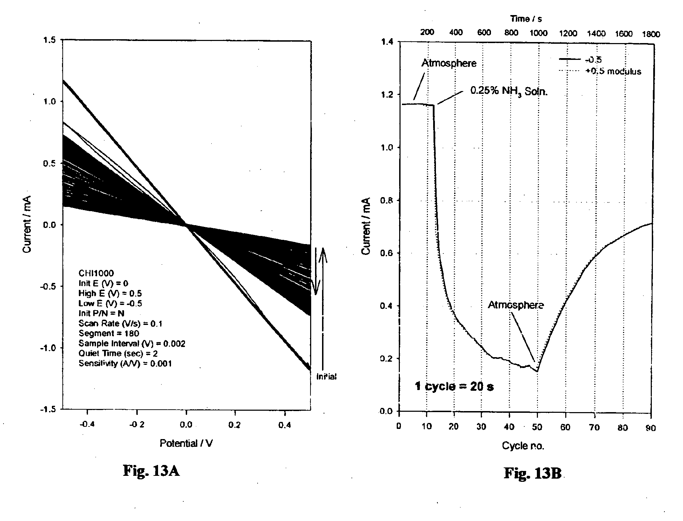

Figure 13 shows the response of the PANI-IDA electrode when exposed to ammonia vapour. Figure 13a shows the current-potential raw data plot while figure 13b shows the current-time response obtained when the current is sampled at the indicated potentials. Initially, under atmospheric conditions, the stable Ohmic plot is obtained. On exposure to ammonia, the conductivity of the polyaniline rapidly decreases - as evidenced by the decreasing slope in Figure 13a and the drop in sampled current in Figure 13b. Once the ammonia is removed, the electrode slowly recovers.

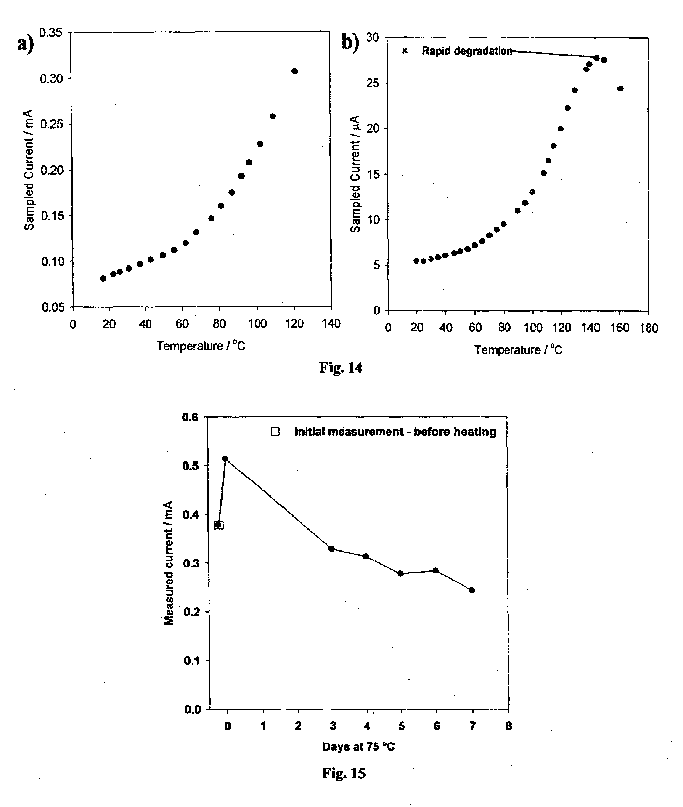

The effect of ambient temperature on the electrode response is given in Figure 14. The current measured for the PANI-IDA is seen to display a positive coefficient with temperature for the range 20 to 120°C - implying that the inherent conductivity of the polyaniline nanoparticles is increasing with temperature. As can be seen for Figure 14a, the electrode displays good thermal stability over short time scales for temperatures up to approximately 120°C. This effect is reversible with the measured current returning to base as the temperature drops. At temperatures over 140°C, an irreversible drop in measured current is observed as the polyaniline undergoes thermal degradation. This drop in conductivity accelerates with increasing temperature.

The long term effects of temperature exposure are given in Figure 15. In this case a PANI-IDA was subjected to a temperature of 75°C over seven days. The PANI-IDA was removed from the oven and allowed cool to room temperature prior to the measurement being taken. On the first day, a sharp increase in measured current is observed before and after heating. For freshly prepared PANI-IDAs, exposure to 75°C for 30 minutes was found to result in a noticeable increase in the measured current, 36% in this case (generally between 25 - 50%). As noted previously, this was employed as a curing method, performed after PANI-IDA preparation. Over the following days a gradual drop in measured current was observed - though the PANI-IDA remains functional after this time. PANI-IDAs stored at room temperature show a similar drift, though over a much longer timescale and retain functionality for 6 months and longer.

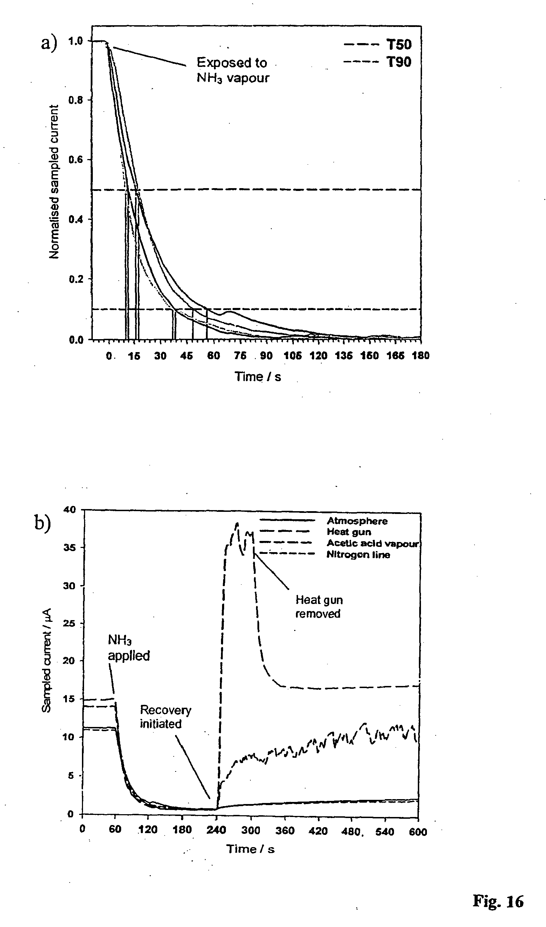

Response time of the electrode has been observed to be fairly rapid. Figure 16a displays the response obtained for a 25 layer inkjet-printed PANI-IDA electrode when exposed to the vapour of 0.25 % ammonia solution (∼60 ppm). From 4 replicates, it can be seen that the T50 (the time required to come to 50% of the final volume) and T90 (the time required to come to 90% of the final volume) of the sensor fall well within the 90s response required by the Instrument Society of America. In Figure 13 it can be noted that the sensor does not recover as rapidly. To this end a number of different methods of enhancing the sensor recovery were investigated. In Figure 16b, the results of the different techniques are present along with normal recovery in atmosphere. By far the best method of recovery was found to be applying a stream of heated air across the electrode via heat gun. The heated air may be applied above the sensor. Alternatively, the heated air may be applied directly to the sensor. It can be seen that the measured current more than doubles on application of the heat before returning to the initial state after the heat gun was removed - in agreement with the data presented in Figure 14. With this method, recovery times <90 s could be achieved. It should be noted that oven heating had a similar effect - rapid recovery of electrode response.

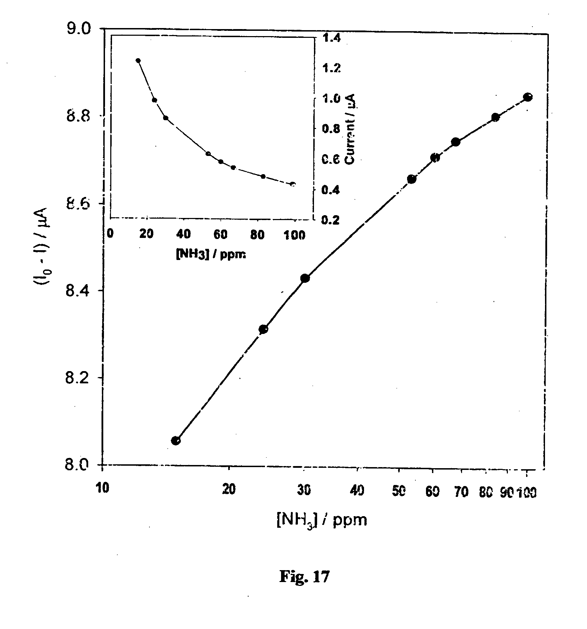

Figure 17 shows the results of headspace analysis performed on a 25 inkjet layered PANI-IDA. In this study, the PANI-IDA was placed within the gas jar with a commercially available ammonia sensor (Impulse XP, Honeywell Analytics). As the ammonia concentration within the gas jar was built up, the response of the PANI-IDA and Impulse XP were noted. The inset of Figure 17 shows the current drop recorded for the PANI-IDA as the concentration of ammonia increases. The main plot shows the results obtained when the difference of measured and initial current (9.23 µA, in this case) is plotted against the log of ammonia concentration. The initial current, I₀, is the value measured with no ammonia present. A quasi-linear response is obtained with an increasing deviation observed at greater concentrations. The large drop in measured current observed from 0 to 15 ppm ammonia (9.23 to 1.23 µA) tends to imply concentrations below this level should be observable.

### Application of inkjet patterned films in a sensing system for hydrogen peroxide (methodology according to Example 4C)

Accurate sensing of hydrogen peroxide is also important in many fields. It is used in many industrial applications as an oxidising, bleaching and sterilising agent. It is also a waste product in atomic power stations. The Printed Circuit Board (PCB) manufacturing industry employs etching baths based on sulphuric acid for various production processes, such as the manufacture of through-hole plating for multiplayer PCBs (black-hole method). In most cases, the etching baths consist of a hydrogen peroxide component of about 25 g/l. The concentration of hydrogen peroxide, in particular, is decisive for the quality of the product, and should be kept as nearly constant as possible. Depending on the number of boards being processed, the hydrogen peroxide concentration in the etching bath diminishes as a result of dispersal and decomposition. The Jumo Corrotrode is a potentiometric sensor that is marketed for this niche market and functions as a hydrogen peroxide detector.

Over the last twenty years, hydrogen peroxide has become the most important bleaching agent in textile industry. This growth founding the use of hydrogen peroxide has also taken place in other industrial applications, because of the favourable ecological properties of hydrogen peroxide (reaction products are oxygen and water) compared to commonly used bleaching products like NaOCl and NaClO₂. The quality of the bleached product is strongly dependent on the concentration of hydrogen peroxide. The concentration of hydrogen peroxide is generally determined off-line, after bleaching, by using optical techniques. An insufficient concentration of hydrogen peroxide may cause insufficient bleaching effects and lowers the quality and colour fastness of the textile dyeing process, that often comes after bleaching. On the other hand, excess concentrations of hydrogen peroxide cause degradation of the textile structure itself.

Russell Mainstreams market a potentiometric hydrogen peroxide probe sensor (WP7 hydrogen peroxide probe) suitable for water disinfection applications. This sensor is resistant to chemicals and surface agents due to a special membrane system protecting the sensor. The sensitivity of the sensor is in the range of 0 to 2000 ppm hydrogen peroxide however the response time of the sensor is slow with a T90 (time required to come to 90 % of final value) of approximately 4 minutes; this response time falls outside the 90s response time required by the Instrument Society of America.

Another market that demands H₂O₂ analysis is the clinical field, where exhaled breath is often monitored for H₂O₂. Hydrogen peroxide in the breath is indicative of lung diseases such as asthma and chronic obstructive pulmonary diseases. Up to now, a common method to collect the exhaled breath condensate is to use a special cooling collector including a freezing cooling tube (usually cooled using ice or liquid nitrogen) and cooling machine, which has a refrigerator's circuit. Once collected, the condensate is analyzed off-line by techniques, such as spectroscopy, with assistance of peroxidase. H₂O₂ is also the most valuable marker for oxidative stress, recognized as one of the major risk factors in progression of disease-related pathophysiological complications in diabetes, atherosclerosis, renal disease, cancer, aging and other condition. Hydrogen peroxide sensors are also needed for the development of biosensors based on enzyme oxidases. Universal Sensors market an amperometric peroxide electrode based on a platinum electrode for these types of applications at a cost of approximately $630 USD.

Other applications of hydrogen peroxide sensing include (http://www.h2o2.com/intro/overview.html):
***Odour control** -* Oxidises hydrogen sulfide, mercaptans, amines and aldehydes. H₂O₂ may be applied directly to aqueous wastes containing these odorants, or to wet scrubbers used to remove them from airstreams.
***Corrosion control -*** destroys residual chlorine and reduced sulphur compounds thiosulphates, sulphites, and sulphides) which form corrosive acids when condensed onto processing equipment and oxidised by air.
***Biological Oxygen Demand (BOD) and Chemical Oxygen Demand (COD) removal -*** Oxidizes both organic and inorganic pollutants which contribute to BOD and COD - H₂O₂ may be needed to oxidize the more resistant substances. H₂O₂ may also affect BOD/COD removal by enhancing the performance of other processes.
***Inorganic oxidation -*** Oxidizes cyanides, NOx/SOx, nitrites, hydrazine, carbonyl sulphide, and other reduced sulphur compounds mentioned above (odour/corrosion control).
***Organic oxidation -*** Hydrolyses formaldehyde, carbon disulfide, carbohydrates, organophosphorous and nitrogen compounds, and various water-soluble polymers; and (with catalysis) destroys phenols, Benzene, Toluene, Ethylbenzene and Xylene (BTEX) pesticides, solvents, plasticizers, chelants, and virtually any other organic requiring treatment.
***Metals oxidation -*** Oxidises ferrous iron, manganese, arsenic, and selenium to improve their adsorption, filtration, or precipitation from process waters and wastewaters.
***Toxicity reduction*/*Biodegradability improvement -*** With catalysis, chemically digests complex organics into smaller, less toxic and more biodegradable fragments. ***Disinfection*/*Bio-control* -** Checks excess biogrowth in water supplies and cooling circuits, and (with catalysis) disinfects process waters and biological effluents.
***Groundwater remediation***
***Industrial wastewater treatment***
***Water*/*wastewater treatment***
***Mouthwash***
***Aseptic food packaging***

Each of the applications mentioned above requires monitoring of hydrogen peroxide at some level. However, there is not sufficient technology available for generating specific, portable peroxide sensors for many of these types of applications.

Extensive studies have been performed with bulk polymer conducting films composed of polyaniline. Such films are extremely useful materials and may be used as a modified electrode material as the films can act as mediatorless electron transfer layers. One process where such use is particularly useful is in the area of biosensing where the conducting polymer can efficiently link enzymes to electrodes through a process referred to as direct electron transfer. Such a linking process may allow many reactions to take place that would, otherwise be thermodynamically unfavourable without a biological species. For example, hydrogen peroxide reduction. In the presence of an enzyme this can take place at relatively low potentials, e.g., -0.1 V vs. Ag/AgCl. One phenomenon that has not been shown to be possible on these bulk films has been the significant direct reduction of hydrogen peroxide. However, using the nanoparticles created from this bulk material, its behaviour changes dramatically.

The phenomenon was demonstrated with electrodes comprised of nanoparticles of polyaniline deposited (by inkjet printing (see Example 3) or other means) onto screen printed carbon-paste electrodes (Example 2A). The significantly enhanced sensing ability we are seeing from these materials maybe as a result of the nanostructuring. This may be attributed to the significant increases in the surface area brought about by the material.

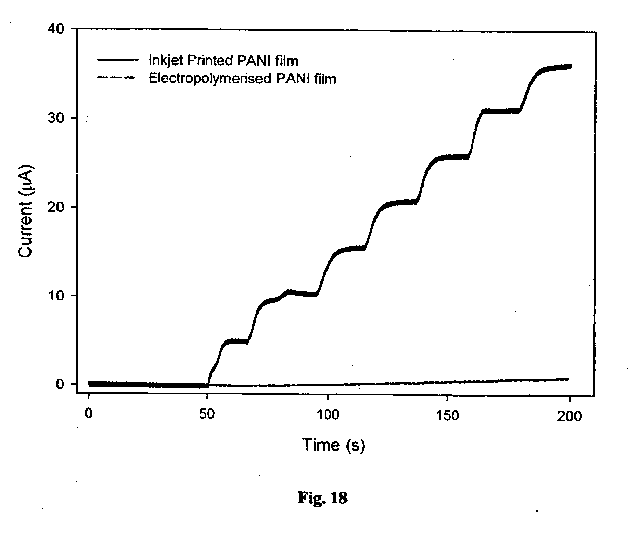

Using the inkjet printing technique of Example 3, polyaniline nanoparticle films were prepared using 20 prints. These modified electrodes were studied for their sensing properties towards H₂O₂. Amperograms were recorded for the reduction of H₂O₂ at -100 mV vs Ag/AgCl in phosphate buffer, pH 6.8 (Example 4C). This was compared to the same process at an electropolymerised bulk polyaniline film (fabricated according to Grennan et al., 2001). These amperograms on the different polyaniline materials was compared in Figure 18. The current observed for H₂O₂ reduction was significantly higher for the inkjet printed nanoparticulate thin film than that observed on bulk polymer (approx. 100 fold). This significant increase in the current observed for H₂O₂ reduction at the nanoparticulate films was attributed to the higher surface area to volume ratio of these materials along with their good electrical properties compared with bulk polymer.

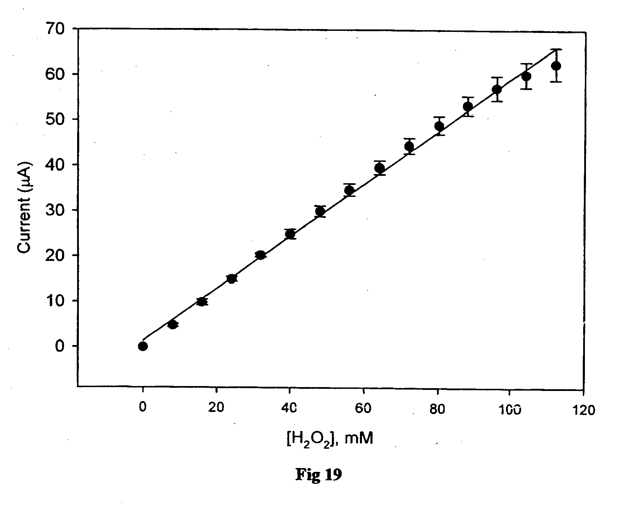

Figure 19 shows the amperometric responses for the inkjet printed films towards H₂O₂, where the sensitivity was found to be 0.5419 µA mM⁻¹ and the detection limit was found to be 1.146 × 10⁻³ M based on a signal-to-noise ratio of 3. The linear range was found to span over three orders of magnitude, from 8 × 10⁻³ - 1.12 × 10⁻¹ M (r = 0.995, n = 3). To the author's knowledge, this appears to be the first report of a H₂O₂ sensor with linearity over this range. The analytical parameters reported here are suitable for application in a glucose biosensor, where H₂O₂ can be exploited for glucose detection as it is a stoiciometrically equivalent product of catalysis of glucose by glucose oxidase (GOD). The clinical range for glucose detection in commercial products is between 1 × 10⁻³ M and 3.5 × 10⁻² M (Wang et al., 2005), which falls within the linear range of this peroxide sensor. In terms of reproducibility, it was found that the relative standard deviation (RSD) of the inkjet printed nanoparticulate polyaniline sensor was 2.13% for nine successive measurements of 8 × 10⁻³ M H₂O₂.

### Application of inkjet patterned films in biosensing applications (methodology according to Example 4D)

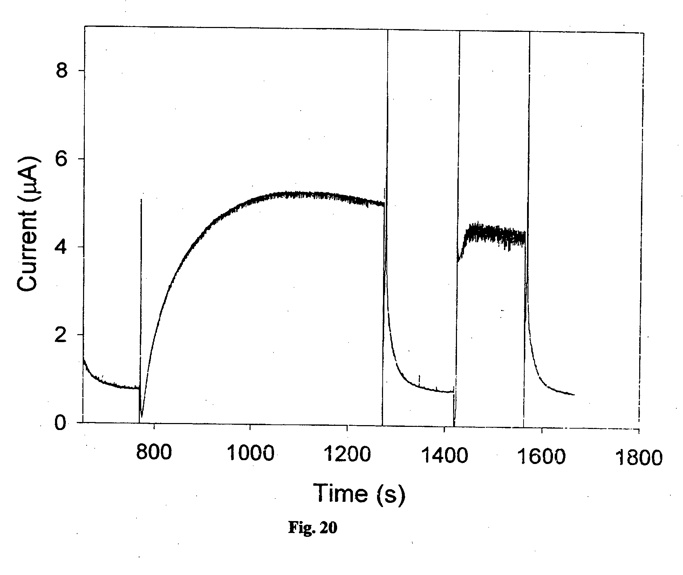

In order to demonstrate the suitability of the polyaniline nanoparticle inkjet printed films towards biosensing, an amperometric assay for free horseradish peroxidase (HRP) was carried out. This was done according to the Example 4D, where polyaniline nanoparticles (Example 1) were inkjet printed (Example 3) onto carbon-paste screen-printed electrodes (Example 2C). HRP can bind directly to polyaniline and when bound, the enzyme (HRP) catalyzes the reduction of H₂O₂, which results in an increase in the HRP catalytic signal (Morrin et al., 2003). This is provided that the polyaniline film can behave as an effective charge transfer mediator between bound HRP and the electrode surface. Figure 20 demonstrates that the inkjet printed films are capable of charge transfer mediation between bound, catalyzed HRP and the electrode surface. The figure shows the binding curve of HRP to the electrode surface in the presence of H₂O₂. The binding if HRP is confirmed as a permanent one by the replacement of buffer with H₂O₂, subsequent to the HRP binding event. Enzymatic catalytic signal is restored showing that the catalytic signal was purely due to bound, rather than free HRP. This could also be theoretically possible for enzymes such as urease or creatinine iminohydrolase, that catalyse their respective substrates, urea and creatinine, to produce ammonia. Given the demonstration of the sensitivity of polyaniline nanoparticle films to ammonia, low-cost biosensors for such analytes is also possible.

### Application of inkjet patterned films in immunosensing applications

Immunoassays use the specific antigen-antibody complexation for analytical purposes. Enzyme immunoassays are well established in clinical diagnostics. For the development of hand-held devices which can be used for point of care measurements, electrochemical immunoassays are promising alternatives to existing immunochemical tests. Moreover, for opaque or optically dense matrices electrochemical methods are superior. Competitive and non-competitive electrochemical immunoassays have been developed often with enzymes as labels using conducting polymer as the diffusionless mediator.

The patterned polyaniline films may be used as an enzyme-based immunosensor in the following theoretical situation:

A detection antibody is immobilized to the polyaniline surface in the first instance. This surface is then exposed to an unknown concentration of antigen of interest and an enzyme- (e.g., HRP) labeled antigen. Both of these species are allowed to bind to the antibody immobilized to the surface of PANI. All excess (unbound) material is then removed and the surface of PANI is exposed to substrate (e.g., H₂O₂) which is catalysed at the surface by the bound enzyme-labeled antigen (HRP). The polyaniline nanoparticle inkjet printed film would be used as a direct electron transfer mediator in this instance (rather than using a soluble mediator). The amount of electrochemical signal generated would be inversely proportional to the amount of 'free' unknown antigen (Figure 21).

Grennan et al. (2003) reported a regeneration-free, multi-calibrant strategy in an immunoassay on electropolymerized polyaniline films. HRP was employed as a label for the assay and was catalysed by H₂O₂. In order to demonstrate the suitability of these inkjet printed films towards this more sophisticated type of approach, free HRP was used again as the biological molecule to be measured. Figure 22 shows the changes in slope of the amperometric signal as different concentrations of HRP are passed over the electrode surface for short times. This shows that these inkjet patterned polyaniline nanoparticle films are very sensitive towards low concentrations (mg ml⁻¹) of HRP, and therefore may hold promise in an immunosensor approach, with their major advantage being their ease of fabrication.

A method to pattern high quality conducting polymer films on conducting substrates by exploiting the nanoparticulate form of the polymer for inkjet printing is illustrated. Aqueous dispersions of polyaniline nanoparticles were inkjet printed onto PET-based screen-printed carbon electrodes (Example 2A). The resulting films adhered well to the electrode surface, and electrochemistry was performed in aqueous media with no effect on the film stability. The polyaniline films had well-defined, reversible electrochemistry in acidic media and their use is described in two applications: direct ammonia sensing and a biosensing application using HRP. The results indicate that the conducting polymer nanoparticles have a wide range of applications in areas such as chemical and biosensing.

While the description specifically refers to polyaniline it will be appreciated that any suitable conducting polymer material may be used. Examples of other conducting polymers are polyacetylenes, polydiacetylenes, polyparaphenylenes, polypyrroles, polythiophenes, polybithiophenes, polyisothiophenes, polyphenylenesulphides and polyanilines. A more comprehensive list is given in Handbook of organic conductive molecules and polymers / edited by Hari Singh Nalwa (1997).

### References

Ballarin, B., Fraleoni-Morgera, A., Frascaro, D., Marazzita, S., Piana, C., Setti, L. (2004). Thermal inkjet microdeposition of PEDOT:PSS on ITO-coated glass and characterization of the obtained film. Synth. Met., 146:201-205.
Calvert, P. (2001). Inkjet printing for materials and devices. Chem. Mater., 13:3299-3305.
Chen, B., Cui, T., Liu, Y., Varahramyan, K. (2003). All-polymer RC filter circuits fabricated with inkjet printing technology. Solid-State Electron., 47:841-847.
Fujii A., Mizukami H., Hashimoto Y., Umeda T., Nishihara Y., Ozaki M., Yoshino K. (2005). Highly efficient photovoltaic cells composed of interpenetrating conducting polymer/C60 heterojunction. Synth. Met., 152:121-124.
Grennan, K., Hansen, C., Cafolla, A., Killard, A.J., Smyth, M.R. (2005). Optimisation and characterisation of biosensors based on polyaniline. Talanta, In Press.
Grennan, K., Strachan, G., Porter, A.J., Killard, A.J., Smyth, M.R. (2003). Atrazine Analysis Using an Amperometric Immunosensor Based on Single-Chain Antibody Fragments and Regeneration-Free Multi-Calibrant Measurement. Anal. Chim. Acta., 500:287-298.
Grennan, K., Killard, A.J., Smyth, M.R. (2001). Physical characterisation of an Amperometric Immunosensor System. Electroanal., 13:745-750.
Huang J., Kaner R.B. (2006). The intrinsic nanofibrillar morphology of polyaniline. Chem. Comm., (4): 367-376.
Killard, A.J., Micheli, L., Grennan, K., Franek, M., Kolar, V., Moscone, D., Palshetti, I., Smyth, M.R. (2001). An Amperometric Separation-Free Immunosensor for Real-Time Environmental Monitoring. Anal. Chim. Acta, 427,173-180.
Le, H.P. (1998). Progress and Trends in Ink-jet Printing Technology. Journal of Imaging Science and Technology., 42:49-62.
Li, G., Martinez, C., Semancik, S. (2005). Controlled electrophoretic patterning of polyaniline from a colloidal suspension. J. Am. Chem. Soc., 127:4903-4909.
Mabrook, M.F., Pearson, C., Petty, M.C. (2005). An inkjet printed chemical fuse. App. Phys. Lett., 86.
Magdassi, S., Ben Moshe, M. (2003). Patterning of organic nanoparticles by ink-jet printing of microemulsions. Langmuir., 19:939-942.
Morrin, A., Wilbeer, F., Ngamna O., Killard A.J., Moulton S.E., Smyth M.R., Wallace G.G. (2005). Novel biosensor fabrication methodology based on processable conducting polyaniline nanoparticles. Electrochem. Comm., 7 (3) 317-322.
Morrin A., Guzman A., Killard A.J., Pingarron J.M., Smyth M.R. (2003). Characterisation of horseradish peroxidase immobilisation on an electrochemical biosensor by colorimetric and amperometric techniques. Biosens. Bioelectron., 18 (5-6) 715-720.
Ngamna, O., Morrin, A., Moulton, S.E., Killard, A.J., Smyth, M.R., Wallace, G.G. (2005). An HRP based biosensor using water-soluble sulphonated polyaniline. Synth. Met., 153(1-3): 185-188.
Paul, K., Wong, W.S., Ready, S., Street, R. (2003). Additive jet printing of polymer thin film resistors. App. Phys. Lett., 83:2070-2072.
Setti, L., Morgera, A., Ballarin, B., Filippini, A., Frascaro, D., Piana, C. (2005). An amperometric glucose biosensor prototype fabricated by thermal inkjet printing. Biosens. Bioelectron., 20:2019-2026.
Sirringhaus, H., Kawase, T., Friend, R.H., Shimoda, T., Inbasekaran, M., Wu, W., Woo, E.P. (2000). High-resolution inkjet printing of all-polymer transistor circuits. Science, 290:21232-126.
Saxena V., Malhotra, B. D. (2003). Prospects of conducting polymers in molecular electronics. Current Applied Physics, 3:293-305.
Yang, Y. (2003). Patent No: US 6,576,975. 'Organic semiconductor devices using inkjet printing technology and device and system employing same'.
Wang, J., Carmon., K.S., Luck, L.A., Sunia, I.I (2005). Electrochemical impedance biosensor for glucose detection utilising a periplasmic E. coli receptor protein. Electrochem. Sol. St. Lett., 8 (8): H61-H64.
Zhang Y., Guan Y., Liu J., Xu J., Cao (2002). Fabrication of covalently attached conducting multilayer self-assembly film of polyaniline by in situ coupling reaction. Synth. Met., 128:305-309.

## Claims

1. Use of an electrode having nanoparticles of polyaniline inkjet printed thereon as an electrochemical sensor for the detection of ammonia.

2. Use as claimed in claim 1 wherein the nanoparticles are spherical in shape.

3. Use as claimed in claim lor 2 wherein the size distribution of the nanoparticles is in the range of from 1nm to 100nm.

4. Use as claimed in any of claims 1 to 3 wherein the size distribution of the nanoparticles is in the range of from 10nm to 50nm.

5. Use as claimed in any of claims 1 to 3 wherein the size distribution of the nanoparticles is in the range of from 20 to 80nm.

6. Use as claimed in any of claims 1 to 3 wherein the size distribution of the nanoparticles is in the range of from 30nm to 80nm.

7. Use as claimed in any of claims 1 to 6 wherein the nanoparticles are inkjet printed onto the electrode using piezoelectric technology.

8. Use as claimed in any of claims 1 to 7 wherein the electrode is a screen printed electrode.

9. Use as claimed in claim 8 wherein the electrode is a carbon paste screen printed electrode.

10. Use as claimed in claim 9 for detecting ammonium ions.

11. Use as claimed in claim 8 wherein the electrode is an interdigitated array.

12. Use as claimed in claim 11 for detecting ammonia in the gas phase.

13. Use as claimed in any of claims 1 to 12 for direct sensing of ammonia.

14. Use as claimed in any of claims 1 to 13 wherein the electrode is a thermostable material, the sensor may undergo dynamic recovery upon the application of heat.

15. Use as claimed in any of claims 1 to 14 for the detection of ammonia in a refrigerated space.

16. Use as claimed in any of claims 1 to 14 for the detection of ammonia in breath analysis.

## Patentansprüche

1. Verwendung einer Elektrode, die Nanopartikel von Polyanilin aufweist, die darauf Inkjet-gedruckt sind, als elektrochemischen Sensor für den Nachweis von Ammoniak.

2. Verwendung nach Anspruch 1, worin die Nanopartikel kugelförmig sind.

3. Verwendung nach Anspruch 1 oder 2, worin die Größenverteilung der Nanopartikel im Bereich von 1 nm bis 100 nm liegt.

4. Verwendung nach einem der Ansprüche 1 bis 3, worin die Größenverteilung der Nanopartikel im Bereich von 10 nm bis 50 nm liegt.

5. Verwendung nach einem der Ansprüche 1 bis 3, worin die Größenverteilung der Nanopartikel im Bereich von 20 nm bis 80 nm liegt.

6. Verwendung nach einem der Ansprüche 1 bis 3, worin die Größenverteilung der Nanopartikel im Bereich von 30 nm bis 80 nm liegt.

7. Verwendung nach einem der Ansprüche 1 bis 6, worin die Nanopartikel auf die Elektrode unter Verwendung von piezoelektrischer Technologie Inkjet-gedruckt werden.

8. Verwendung nach einem der Ansprüche 1 bis 7, worin die Elektrode eine siebgedruckte Elektrode ist.

9. Verwendung nach Anspruch 8, worin die Elektrode eine siebgedruckte Kohlepaste-Elektrode ist.

10. Verwendung nach Anspruch 9 für den Nachweis von Ammoniumionen.

11. Verwendung nach Anspruch 8, worin die Elektrode ein interdigitiertes Array ist.

12. Verwendung nach Anspruch 11 für den Nachweis von Ammoniak in der Gasphase.

13. Verwendung nach einem der Ansprüche 1 bis 12 für die direkte Messung von Ammoniak.

14. Verwendung nach einem der Ansprüche 1 bis 13, worin die Elektrode ein thermostabiles Material ist, der Sensor bei Anwendung von Wärme eine dynamische Erholung erfahren kann.

15. Verwendung nach einem der Ansprüche 1 bis 14 für den Nachweis von Ammoniak in einem Kühlraum.

16. Verwendung nach einem der Ansprüche 1 bis 14 für den Nachweis von Ammoniak in der Atemanalyse.

## Revendications

1. Utilisation d'une électrode sur laquelle des nanoparticules de polyaniline ont été imprimées par jet d'encre comme capteur électrochimique pour la détection de l'ammoniac.

2. Utilisation selon la revendication 1, dans laquelle les nanoparticules ont une forme sphérique.

3. Utilisation selon la revendication 1 ou 2, dans laquelle la distribution granulométrique des nanoparticules est dans la plage de 1 nm à 100 nm.

4. Utilisation selon l'une quelconque des revendications 1 à 3, dans laquelle la distribution granulométrique des nanoparticules est dans la plage de 10 nm à 50 nm.

5. Utilisation selon l'une quelconque des revendications 1 à 3, dans laquelle la distribution granulométrique des nanoparticules est dans la plage de 20 nm à 80 nm.

6. Utilisation selon l'une quelconque des revendications 1 à 3, dans laquelle la distribution granulométrique des nanoparticules est dans la plage de 30 nm à 80 nm.

7. Utilisation selon l'une quelconque des revendications 1 à 6, dans laquelle les nanoparticules sont imprimées par jet d'encre sur l'électrode en utilisant une technologie piézoélectrique.

8. Utilisation selon l'une quelconque des revendications 1 à 7, dans laquelle l'électrode est une électrode sérigraphiée.

9. Utilisation selon la revendication 8, dans laquelle l'électrode est une électrode à pâte de carbone sérigraphiée.

10. Utilisation selon la revendication 9 pour la détection d'ions d'ammonium.

11. Utilisation selon la revendication 8, dans laquelle l'électrode est un réseau interdigité.

12. Utilisation selon la revendication 11 pour la détection de l'ammoniac dans la phase gazeuse.

13. Utilisation selon l'une quelconque des revendications 1 à 12 pour une détection directe de l'ammoniac.

14. Utilisation selon l'une quelconque des revendications 1 à 13, dans laquelle l'électrode est un matériau thermostable et le capteur peut subir une restauration dynamique lors de l'application de chaleur.

15. Utilisation selon l'une quelconque des revendications 1 à 14 pour la détection de l'ammoniac dans un espace réfrigéré.

16. Utilisation selon l'une quelconque des revendications 1 à 14 pour la détection de l'ammoniac lors d'une analyse d'haleine.
